# EUROPÄISCHE PATENTSCHRIFT

(11) **EP 3 048 092 B1**
(45) Veröffentlichungstag und Bekanntmachung des Hinweises auf die Patenterteilung: **12.04.2017**
(21) Anmeldenummer: 15152518.5
(22) Anmeldetag: 26.01.2015
(51) Int. Cl.: C07C 51/23, C07C 53/02

(54) **Verfahren zur katalytischen Erzeugung von Ameisensäure bei einem Sauerstoffpartialdruck unter 1 bar und Regeneration des dabei eingesetzten Katalysators**
Method for catalytically producing formic acid at partial oxygen pressure lower than 1 bar and regeneration of the catalyst involved
Procédé de production catalytique d'acide formique avec une pression partielle d'oxygène inférieure à 1 bar et régénération du catalyseur utilisé pour cela

(43) Veröffentlichungstag der Anmeldung: 27.07.2016
(73) Patentinhaber: Jbach GmbH, 96120 Bischberg (DE)
(72) Erfinder: Jbach, Dr. Hermann Wolf, 96120 Bischberg (DE)
(74) Vertreter: Dr. Gassner & Partner mbB

(56) Entgegenhaltungen:
- EP-B1- 2 473 467
- JP-A- 2008 273 915
- US-A- 5 695 606

## Beschreibung

Die Erfindung betrifft ein Verfahren zur katalytischen Erzeugung von Ameisensäure und Regeneration des dabei eingesetzten Katalysators. Ein derartiges Verfahren ist aus der EP 2 473 467 B1 bekannt. Dabei wird ein als Katalysator dienendes Polyoxometallat-Ion der allgemeinen Formel [PMoₓV_{y}O₄₀]⁵⁻ bei einer Temperatur oberhalb von 70°C und unterhalb von 120°C mit einem alpha-Hydroxyaldehyd, einer alpha-Hydroxycarbonsäure, einem Kohlenhydrat oder einem Glycosid in einer flüssigen Lösung in einem Gefäß in Kontakt gebracht, wobei 6 < x < 11 und 1 < y < 6 und x + y = 12, wobei x und y jeweils eine ganze Zahl ist. Der dabei reduzierte Katalysator wird durch Oxidation wieder in seinen Ausgangszustand versetzt.

Gemäß der EP 2 473 467 B1 ist es vorteilhaft, wenn das Inkontaktbringen unter einem Sauerstoffpartialdruck von 1 bis 500 bar erfolgt. Je höher der Sauerstoffpartialdruck ist, desto schneller erfolgt die Oxidation des bei dem Verfahren reduzierten Katalysators. Es hat sich nun jedoch gezeigt, dass zur Durchführung des Verfahrens eingesetzte Druckgefäße aus Stahl bei der Durchführung des Verfahrens stark korrodieren. Um dies zu vermeiden, können hochkorrosionsbeständige, jedoch verhältnismäßig teure Nickel-Chrom-Molybdän-Legierungen, wie z. B. das sogenannte "Hastelloy C" der Firma Haynes International, Inc., Kokomo, USA, verwendet werden. Wenn das Gefäß aus einer solchen Legierung hergestellt wird, wird dadurch die Durchführung des Verfahrens verhältnismäßig teuer. Aufgabe der vorliegenden Erfindung ist es daher, eine kostengünstigere Möglichkeit zur Durchführung des Verfahrens anzugeben.

Die Aufgabe wird durch die Merkmale des Patentanspruchs 1 gelöst. Zweckmäßige Ausgestaltungen ergeben sich aus den Merkmalen der Patentansprüche 2 bis 13.

Erfindungsgemäß ist ein Verfahren zur katalytischen Erzeugung von Ameisensäure bei einem Sauerstoffpartialdruck unter 1 bar und der Regeneration des dabei eingesetzten Katalysators vorgesehen, wobei ein als Katalysator dienendes Polyoxometallat-Ion der allgemeinen Formel [PMoₓV_{y}O₄₀]ⁿ⁻ bei einer Temperatur oberhalb von 70°C und unterhalb von 120°C mit einem alpha-Hydroxyaldehyd, einer alpha-Hydroxycarbonsäure, einem Kohlenhydrat, einem Glycosid oder einem eine Kohlenstoffkette enthaltenden Polymer mit mindestens einer wiederholt als Substituent an die Kohlenstoffkette gebundenen OH-Gruppe und/oder mit einem wiederholt in der Kohlenstoffkette enthaltenen O-, N- oder S-Atom in einer flüssigen Lösung in einem Gefäß in Kontakt gebracht wird, wobei 6 ≤ x ≤ 11 und 1 ≤ y ≤ 6 und 3 < n < 10 und x + y = 12, wobei n, x und y jeweils eine ganze Zahl ist. Der dabei reduzierte Katalysator wird durch Oxidation wieder in seinen Ausgangszustand versetzt. Im Sinne der Erfindung wird unter einem Katalysator also auch ein Stoff verstanden, der während des Verfahrens durch Reduktion verändert und durch Oxidation wieder in seinen Ausgangszustand versetzt wird. Das Inkontaktbringen in dem Gefäß erfolgt bei einem Sauerstoffpartialdruck unter 1 bar. Zur Oxidation des Katalysators wird ein Teil der flüssigen Lösung aus dem Gefäß ausgeleitet, mit Sauerstoff oder einem Sauerstoff enthaltenden Gasgemisch bei einem Sauerstoffpartialdruck von 1 bis 500 bar beaufschlagt und anschließend wieder dem Rest der flüssigen Lösung zugeführt. Dabei wird der Sauerstoffpartialdruck in dem Gefäß stets unter 1 bar gehalten. Der den Teil der flüssigen Lösung beaufschlagende Sauerstoffpartialdruck wird von 1 bis 500 bar auf unter 5 bar herabgesetzt, bevor der Teil der flüssigen Lösung wieder dem Rest der flüssigen Lösung zugeführt wird.

Es ist auch möglich, dass der unter 1 bis 500 bar Sauerstoffpartialdruck stehende Teil der flüssigen Lösung unter diesem Druck oder unter nur teilweise reduziertem Druck wieder dem Rest der flüssigen Lösung zugeführt wird. Dabei entgast der zugeführte Teil der flüssigen Lösung wegen des geringeren Drucks, mit dem der Rest der flüssigen Lösung beaufschlagt ist. Um zu verhindern, dass es zu einem Druckanstieg auf 1 bar oder darüber kommt, muss das aus der Flüssigkeit austretendes Gas in ausreichender Menge aus dem Gefäß entweichen können. Das Zuführen des unter 1 bis 500 bar Sauerstoffpartialdruck oder unter teilweise reduziertem Druck stehenden Teils der flüssigen Lösung unter diesem Druck zum Rest der flüssigen Lösung kann wegen der Druckdifferenz zwischen dem Teil und dem Rest der flüssigen Lösung auch als ein Einströmen des Teils in den Rest der flüssigen Lösung gestaltet und zu einer Durchmischung der flüssigen Lösung genutzt werden. Das Einströmen kann über entsprechend gestaltete, insbesondere in der Strömungsrichtung und/oder in ihrem Auslassdurchmesser variierbare, Düsen erfolgen. Durch Veränderung des Auslassdurchmessers kann die Ausströmgeschwindigkeit des Teils der flüssigen Lösung aus der Düse verändert werden.

Bei dem Sauerstoff enthaltenden Gasgemisch kann es sich beispielsweise um Luft handeln. Das Gasgemisch kann mehr als 10 Vol.-% Sauerstoff enthalten. Das Beaufschlagen mit dem Sauerstoff oder dem Gasgemisch kann z. B. durch Einblasen des Sauerstoffs oder des Gasgemischs in den Teil der flüssigen Lösung erfolgen.

Die Lösung kann ein Lösemittel umfassen. Das Vorsehen eines Lösemittels ist nicht erforderlich, wenn das alpha-Hydroxyaldehyd, die alpha-Hydroxycarbonsäure, das Kohlenhydrat, das Glycosid oder das Polymer bereits in flüssiger Form vorliegt. Unter einem alpha-Hydroxyaldehyd wird jedes Molekül verstanden, bei welchem eine OH-Gruppe direkt an einem C-Atom gebunden ist, wobei an dem C-Atom auch direkt das C-Atom einer Aldehyd-Gruppe gebunden ist. Unter einer alpha-Hydroxycarbonsäure wird jedes Molekül verstanden, bei welchem eine OH-Gruppe direkt an einem C-Atom gebunden ist, wobei an dem C-Atom auch direkt das C-Atom einer Carboxy-Gruppe gebunden ist. Unter einem alpha-Hydroxyaldehyd und unter einer alpha-Hydroxycarbonsäure kann auch jede Substanz verstanden werden, welche ein alpha-Hydroxyaldehyd oder eine alpha-Hydroxycarbonsäure enthält.

Die Erfinder der vorliegenden Erfindung haben erkannt, dass die Korrosion durch das Zusammenwirken des erhöhten Sauerstoffpartialdrucks und der Temperatur oberhalb von 70°C und unterhalb von 120°C stark gefördert wird. Sie haben weiterhin erkannt, dass es für die effektive Oxidation des reduzierten Katalysators nicht erforderlich ist, dass die Beaufschlagung mit Sauerstoff oder einem Sauerstoff enthaltendem Gasgemisch bei einem Sauerstoffpartialdruck von 1 bis 500 bar bei einer Temperatur oberhalb von 70°C und unterhalb von 120°C erfolgt.

Das erfindungsgemäße Verfahren ermöglicht es, den Teil der flüssigen Lösung abzukühlen und dann dem erhöhten Sauerstoffpartialdruck auszusetzen, so dass dadurch ein den Teil der flüssigen Lösung beim Beaufschlagen mit dem Sauerstoff oder dem Gasgemisch kontaktierendes weiteres Gefäß keiner stark erhöhten Korrosion unterliegt. Alternativ kann auf das Abkühlen auch verzichtet werden und nur ein verhältnismäßig kleines weiteres Gefäß zur Beaufschlagung mit dem Sauerstoff oder dem Gasgemisch genutzt werden. Die verhältnismäßig hohen Kosten für ein hochkorrosionsbeständiges Material fallen für das verhältnismäßig kleine weitere Gefäß nicht so sehr ins Gewicht wie bei einer Bereitstellung des verhältnismäßig großen Gefäßes, in dem die katalytische Umsetzung mit dem alpha-Hydroxyaldehyd, der alpha-Hydroxycarbonsäure, dem Kohlenhydrat oder dem Glycosid erfolgt, aus einem solchen hochkorrosionsbeständigen Material. Das Gefäß, in dem die katalytische Umsetzung erfolgt, kann aus einem sehr günstigen Material hergestellt werden. Es kann sogar nach oben hin offen sein, weil die katalytische Umsetzung auch bei Atmosphärendruck erfolgen kann. Eine Druckbeständigkeit des Gefäßes ist nicht erforderlich.

Zur Zurückhaltung von in der Flüssigkeit verteilten Feststoffen kann beim Ausleiten des Teils der flüssigen Lösung aus dem Gefäß ein Filter vorgesehen sein. Ein solcher Filter kann verhindern, dass Feststoffe in den in Strömungsrichtung nachgelagerten Teil der Vorrichtung zur Durchführung des Verfahrens gelangen und dort beispielsweise Ventile oder sonstige Teile der Vorrichtung zusetzen oder sonst in ihrer Funktion beeinträchtigen.

Bei einer Ausgestaltung des erfindungsgemäßen Verfahrens liegt das alpha-Hydroxyaldehyd, die alpha-Hydroxycarbonsäure, das Kohlenhydrat, das Glycosid oder das Polymer in der flüssigen Lösung in Form von darin verteilten Feststoffen vor. Die Feststoffe können fein verteilt oder grob verteilt in der flüssigen Lösung vorliegen.

Bei dem Polymer kann es sich um ein Polyester, ein Polyamin oder ein Polyamid, insbesondere Polyhexamethylenadipinsäureamid (Nylon), handeln. Das Polymer kann ein Polymer ohne Weichmacher sein. Weichmacher können die Aktivität des Katalysators negativ beeinflussen.

Alpha-Hydroxyaldehyde, alpha-Hydroxycarbonsäuren, Kohlenhydrate und Glycoside kommen in einer großen Zahl nachwachsender Rohstoffe, wie beispielsweise Stärke, Zellulose oder Hemizellulose vor. Stärke, Zellulose und Hemizellulose fallen in großen Mengen als Produkt aus Ackerpflanzen oder beim industriellen Holzaufschluss, beispielsweise zur Papierherstellung an.

Bei dem alpha-Hydroxyaldehyd, der alpha-Hydroxycarbonsäure, dem Kohlenhydrat oder dem Glycosid kann es sich um ein Monosaccharid, insbesondere einer Aldose, Disaccharid, Oligosaccharid oder Polysaccharid, Stärke, Zellulose, Hemizellulose, Glucose, Saccharose, Xylose, Zellobiose, Xylan, ein Hetereooligosaccharid, ein Heteropolysaccharid, Glycolsäure oder Milchsäure oder einen das alpha-Hydroxyaldehyd, die alpha-Hydroxycarbonsäure, das Kohlenhydrat oder das Glycosid enthaltenden Reststoffen oder, insbesondere nachwachsenden, insbesondere unbehandelten, Rohstoff handeln. Unbehandelt bedeutet dabei, dass der Rohstoff zuvor nicht chemisch aufgeschlossen worden ist. Bei dem Reststoff oder dem nachwachsenden Rohstoff kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage handeln. Das alpha-Hydroxyaldehyd, die alpha-Hydroxycarbonsäure, das Kohlenhydrat oder das Glycosid kann auch ein Gemisch aus mindestens zwei der genannten Substanzen umfassen oder aus mindestens einer der genannten Substanzen oder dem Gemisch entstanden sein, wie dies z. B. bei Braunkohle oder Torf der Fall ist.

Viele der genannten Rohstoffe fallen als Nebenprodukte, beispielsweise bei der Papierherstellung oder Holzverarbeitung an. Sie stehen damit als günstiger Ausgangsstoff für das erfindungsgemäße Verfahren zur Verfügung. Das erfindungsgemäße Verfahren kann dadurch sehr kostengünstig durchgeführt werden. Dazu trägt auch die Oxidation des bei der katalysierten Reaktion reduzierten Katalysators (= Reoxidation) bei, die eine sehr lange Verwendung des Katalysators erlaubt.

Das Inkontaktbringen in dem Gefäß kann bei Atmosphärendruck erfolgen, wobei der Gasdruck in dem Gefäß stets bei Atmosphärendruck gehalten wird. Dadurch kann das Gefäß sehr kostengünstig bereitgestellt werden, weil es keinem erhöhten Druck standhalten muss. Es kann sich dabei beispielsweise um ein Kunststoffgefäß handeln, welches der Temperatur oberhalb von 70°C und unterhalb von 120°C standhält. Die oben genannten Korrosionsprobleme treten bei einem solchen Kunststoffgefäß nicht auf. Unter Atmosphärendruck wird dabei derjenige Luftdruck verstanden, der jeweils bei Durchführung des Verfahrens in der Umgebung des Gefäßes außerhalb eines Gebäudes auf derselben Höhe über dem Meeresspiegel, auf welcher das Verfahren durchgeführt wird, herrscht.

In dem Gefäß kann die Lösung bei einem Sauerstoffpartialdruck von unter 1 bar, insbesondere bei Atmosphärendruck, über Stunden, Tage oder sogar Wochen umgesetzt werden. Es ist nicht erforderlich die Kontaktzeit mit dem Gefäß zeitlich zu limitieren, um eine Korrosion des Gefäßes zu vermeiden.

Um den Gasdruck in dem Gefäß stets bei Atmosphärendruck zu halten kann das Gefäß eine nach außen führende Öffnung aufweisen, beispielsweise indem es nach oben hin einfach offen ist. Um zu verhindern, dass bei einem solchen Gefäß auch entstandene Ameisensäure dampfförmig durch diese Öffnung entweicht, kann das Gefäß an der Öffnung eine Kondensationsvorrichtung aufweisen, an der entstandener Ameisensäuredampf kondensieren kann, so dass ein dadurch entstandenes Kondensat in das Gefäß zurückfließen oder zurücktropfen kann.

Die Oxidation des Katalysators in dem Teil der flüssigen Lösung kann innerhalb von wenigen Minuten oder sogar Sekunden erfolgen. Beispielsweise ist es möglich, den Teil der flüssigen Lösung höchstens 10 Minuten, insbesondere höchstens 5 Minuten, insbesondere höchstens 2 Minuten, insbesondere höchstens 1 Minute, insbesondere höchstens 40 Sekunden, insbesondere höchstens 30 Sekunden, insbesondere höchstens 20 Sekunden, insbesondere höchstens 10 Sekunden, insbesondere höchstens 5 Sekunden, mit dem Sauerstoff oder dem Gasgemisch bei einem Sauerstoffpartialdruck von 1 bis 500 bar zu beaufschlagen.

Der Teil der flüssigen Lösung kann kontinuierlich oder in, insbesondere regelmäßigen, Intervallen aus dem Gefäß ausgeleitet, bei einem Sauerstoffpartialdruck von 1 bis 500 bar oxidiert und anschließend wieder dem Rest der flüssigen Lösung kontinuierlich oder in, insbesondere regelmäßigen, Intervallen zugeführt werden. Auch das Oxidieren kann kontinuierlich oder in, insbesondere regelmäßigen, Intervallen durchgeführt werden. Dies ermöglicht es, den in dem Gefäß enthaltenen Katalysator während des gesamten Verfahrens in ausreichender Menge in oxidierter und damit zur Umsetzung des alpha-Hydroxyaldehyds, der alpha-Hydroxycarbonsäure, des Kohlenhydrats, des Glycosids oder des Polymers geeigneter Form bereitzustellen, ohne dass dazu eine allzu große Menge des verhältnismäßig teuren Katalysators eingesetzt werden muss. Zum Durchführen des Oxidierens in Intervallen ist es beispielsweise möglich, den Teil der flüssigen Lösung in ein weiteres Gefäß einzuleiten, dort den Sauerstoff oder das Gasgemisch in den Teil der flüssigen Lösung einzublasen und den Teil der flüssigen Lösung dann, beispielsweise mittels einer hydraulischen Presse, mit einem Sauerstoffpartialdruck von 1 bis 500 bar für bis zu 5 Minuten zu beaufschlagen. Anschließend kann der Teil der flüssigen Lösung nach Druckentlastung aus dem weiteren Gefäß ausgeleitet und wieder dem Rest der flüssigen Lösung zugeführt werden.

Auch das gesamte Verfahren kann kontinuierlich oder in, insbesondere regelmäßigen, Intervallen, durchgeführt werden. Dazu kann beispielsweise kontinuierlich oder in, insbesondere regelmäßigen, Intervallen ein weiterer Teil der flüssigen Lösung aus dem Gefäß ausgeleitet, darin enthaltene Ameisensäure, beispielsweise in Form eines Formiats, abgesondert und der verbleibende Teil der flüssigen Lösung wieder dem Rest der flüssigen Lösung zugeführt werden. Ein dazu geeignetes Verfahren ist beispielsweise in der bisher unveröffentlichten internationalen

Patentanmeldung PCT/EP2014/074930 beschrieben.

Bei einer weiteren Ausgestaltung des erfindungsgemäßen Verfahrens wird der Teil der flüssigen Lösung vor dem Beaufschlagen mit dem Sauerstoff oder dem Gasgemisch abgekühlt und dem Sauerstoffpartialdruck von 1 bis 500 bar bei einer Temperatur von höchstens 95°C, insbesondere höchstens 85°C, insbesondere höchstens 75°C, insbesondere höchstens 65°C, insbesondere höchstens 55°C, insbesondere höchstens 45°C, insbesondere höchstens 35°C, insbesondere höchstens 25°C, ausgesetzt. Je weiter der Teil der flüssigen Lösung abgekühlt wird und je niedriger die Temperatur ist, bei der der Teil der flüssigen Lösung dem Sauerstoffpartialdruck von 1 bis 500 bar ausgesetzt wird, desto geringer sind die Anforderungen an das Material eines weiteren Gefäßes, in dem die Beaufschlagung mit dem Sauerstoff oder dem Gasgemisch erfolgt, im Hinblick auf dessen Korrosionsbeständigkeit. Das Abkühlen kann mittels eines Wärmetauschers erfolgen, welcher es ermöglicht, die dem Teil der flüssigen Lösung entzogene Wärme dem Teil der flüssigen Lösung zuzuführen, bevor dieser wieder dem Rest der flüssigen Lösung zugeführt wird. Dies kann beispielsweise mit Hilfe einer Wärmepumpe erfolgen.

Der Teil der flüssigen Lösung kann mittels mindestens einer Pumpe, insbesondere einer Dickstoffpumpe, gefördert werden. Das Fördern kann vor und/oder nach der Beaufschlagung mit dem Sauerstoffpartialdruck von 1 bis 500 bar erfolgen. Eine Dickstoffpumpe ermöglicht es, den Teil der flüssigen Lösung zu fördern, ohne dass in der flüssigen Lösung verteilte Feststoffe herausgefiltert werden müssen.

Der Teil der flüssigen Lösung kann dem Sauerstoffpartialdruck von 1 bis 500 bar in einem weiteren Gefäß ausgesetzt werden. Das weitere Gefäß kann dazu verhältnismäßig klein ausgelegt sein. Um den Teil der flüssigen Lösung dem Sauerstoffpartialdruck von 1 bis 500 bar auszusetzen ist beispielsweise ein aus der EP 0090128 A1 bekanntes Verfahren zur Begasung von Flüssigkeit geeignet. Eine andere Möglichkeit der Begasung besteht beispielsweise darin, den Teil der flüssigen Lösung über eine verhältnismäßig große von der Lösung zu benetzende Oberfläche fließen zu lassen und dabei dem Sauerstoff oder dem Sauerstoff enthaltenden Gasgemisch beim Sauerstoffpartialdruck von 1 bis 500 bar auszusetzen. Der Sauerstoff oder das Sauerstoff enthaltende Gasgemisch können dabei im Gegenstrom zur Lösung strömen.

Der den Teil der flüssigen Lösung beaufschlagende Sauerstoffpartialdruck von 1 bis 500 bar kann auf unter 1 bar, insbesondere auf den bei Atmosphärendruck vorliegenden Sauerstoffpartialdruck, herabgesetzt werden, bevor der Teil der flüssigen Lösung wieder dem Rest der flüssigen Lösung zugeführt wird.

Bei einer Ausgestaltung des Verfahrens wird der Sauerstoff oder das Gasgemisch dem Teil der flüssigen Lösung zugeführt, das weitere Gefäß ist ein Gehäuse eines Schneckenverdichters und der Sauerstoffpartialdruck wird dadurch aufgebaut, dass der Teil der flüssigen Lösung zusammen mit dem Sauerstoff oder dem Gasgemisch mittels des Schneckenverdichters verdichtet wird. Der Sauerstoff oder das Gasgemisch können dabei in dem Teil der flüssigen Lösung in Form von Gasblasen enthalten sein. Bei einem Schneckenverdichter handelt es sich um eine Vorrichtung, bei der beispielsweise eine in einem Gehäuse sich drehende Förderschnecke die flüssige Lösung gegen einen im Durchlass begrenzten Auslass presst und dadurch einen Druck auf die Lösung ausübt. Statt einer Förderschnecke können auch zwei ineinandergreifende Förderschnecken in einem Gehäuse vorgesehen sein. Der Schneckenverdichter kann dabei auch die Funktion der Pumpe oder der Dickstoffpumpe erfüllen. Bei einer Ausgestaltung kann der Schneckenverdichter zusätzlich mit einer Zerkleinerungsvorrichtung - ähnlich wie bei einem Fleischwolf - ausgestattet sein. Als Zerkleinerungsvorrichtung kann beispielsweise direkt hinter dem Auslass ein rotierendes Messer vorgesehen sein, welches ggf. in dem Teil der flüssigen Lösung enthaltene Feststoffe zerkleinert, wenn sie aus dem Auslass heraustreten. Dadurch ist gleichzeitig eine Zerkleinerung und ein Aufschluss von den in der Lösung verteilten Feststoffen möglich. Der Schneckenverdichter kann aus einem hochkorrosionsbeständigen Material, wie beispielsweise der oben genannten "Hastelloy C"-Legierung hergestellt sein.

Bei oder nach der Herabsetzung des den Teil der flüssigen Lösung beaufschlagenden Sauerstoffpartialdrucks oder nachdem der Teil der flüssigen Lösung wieder dem Rest der flüssigen Lösung zugeführt worden ist kann Gas, welches aus dem Teil der flüssigen Lösung oder der flüssigen Lösung ausgast, aus einer zur Durchführung des Verfahrens geeigneten Vorrichtung entweichen gelassen oder abgeführt werden. Wenn der Teil der flüssigen Lösung dem Rest der flüssigen Lösung in dem Gefäß zugeführt wird, kann das ausgasende Gas in die Atmosphäre entweichen, wenn das Gefäß dazu zur Atmosphäre hin offen ist oder mit einem Überdruckventil verschlossen ist, welches einen Druckanstieg auf 1 bar und darüber verhindert.

Nachfolgend wird die Erfindung anhand eines Ausführungsbeispiels näher erläutert.

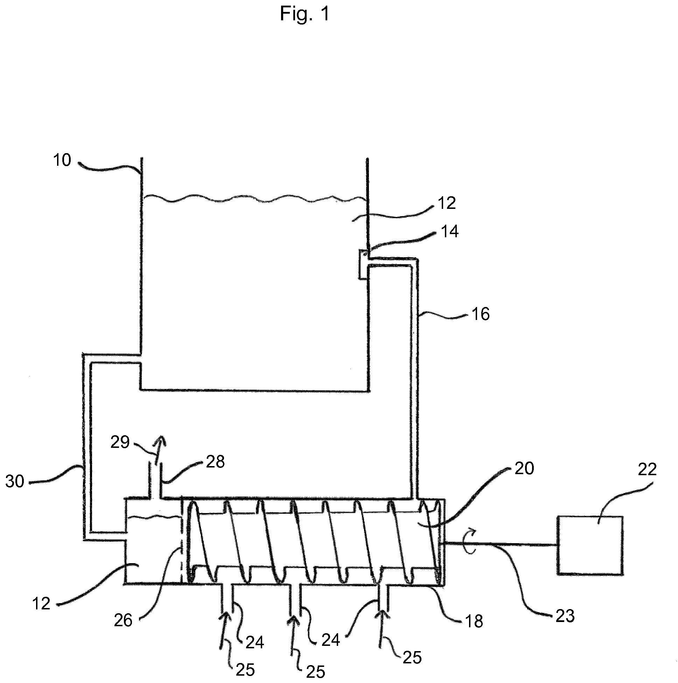

- Figur 1 zeigt: eine schematische Darstellung einer zur Durchführung des Verfahrens geeigneten Vorrichtung.

Die Figur 1 zeigt ein Gefäß 10 mit darin enthaltender flüssiger Lösung 12. Die flüssige Lösung 12 enthält den Katalysator und ein alpha-Hydroxyaldehyd, eine alpha-Hydroxycarbonsäure, ein Kohlenhydrat oder ein Glycosid und ist auf eine Temperatur oberhalb von 70°C und unterhalb von 120°C temperiert. An dem Gefäß 10 ist eine Ableitung 16 mit einem davorgeschalteten Filter 14 zur Ausleitung eines Teils der flüssigen Lösung 12 vorgesehen. Durch die Ableitung 16 gelangt der Teil der flüssigen Lösung 12 in das Gehäuse 18 des Schneckenverdichters. Darin verdichtet die von dem Antrieb 22 über die Antriebswelle 23 angetriebene Verdichterschnecke 20 den in das Gehäuse 18 des Schneckenverdichters eingeleiteten Teil der flüssigen Lösung 12 mit darin über die Luftzufuhrstutzen 24 eingeleiteter Luft 25. Der Teil der flüssigen Lösung 12 wird dabei gegen die Lochmaske 26 gepresst. Dabei baut sich im Schneckenverdichter ein Sauerstoffpartialdruck von deutlich über 1 bar auf. Der Druck ist abhängig vom Widerstand, den die Lochmaske 26 dem Teil der flüssigen Lösung 12 entgegensetzt und der Konzeption des Schneckenverdichters, insbesondere der Steigung der Verdichterschnecke 20 und der Drehzahl, mit welcher die Verdichterschnecke 20 durch den Antrieb 22 gedreht wird. Hinter der Lochmaske 26 sammelt sich der Teil der flüssigen Lösung 12 bei herabgesetztem Sauerstoffpartialdruck an, wobei ausgasendes Gas 29 durch den Entgasungsstutzen 28 entweichen kann. Der Teil der flüssigen Lösung 12 gelangt dann durch die Zuleitung 30 wieder zum Rest der flüssigen Lösung 12 in dem Gefäß 10. Dazu kann der Teil der flüssigen Lösung 12 mittels einer hier nicht dargestellten Pumpe durch die Zuleitung 30 gefördert werden.

### Bezugszeichenliste

- 10: Gefäß
- 12: flüssige Lösung
- 14: Filter
- 16: Ableitung
- 18: Gehäuse eines Schneckenverdichters
- 20: Verdichterschnecke
- 22: Antrieb
- 23: Antriebswelle
- 24: Luftzufuhrstutzen
- 25: Luft
- 26: Lochmaske
- 28: Entgasungsstutzen
- 29: Gas
- 30: Zuleitung

## Patentansprüche

1. Verfahren zur katalytischen Erzeugung von Ameisensäure bei einem Sauerstoffpartialdruck unter 1 bar und Regeneration des dabei eingesetzten Katalysators, wobei ein als Katalysator dienendes Polyoxometallat-Ion der allgemeinen Formel [PMoₓV_{y}O₄₀]ⁿ⁻ bei einer Temperatur oberhalb von 70°C und unterhalb von 120°C mit einem alpha-Hydroxyaldehyd, einer alpha-Hydroxycarbonsäure, einem Kohlenhydrat, einem Glycosid oder einem eine Kohlenstoffkette enthaltenden Polymer mit mindestens einer wiederholt als Substituent an die Kohlenstoffkette gebundenen OH-Gruppe und/oder mit einem wiederholt in der Kohlenstoffkette enthaltenen O-, N- oder S-Atom in einer flüssigen Lösung (12) in einem Gefäß (10) in Kontakt gebracht wird, wobei 6 ≤ x ≤ 11 und 1 ≤ y ≤ 6 und 3 < n < 10 und x + y = 12, wobei n, x und y jeweils eine ganze Zahl ist, wobei der dabei reduzierte Katalysator durch Oxidation wieder in seinen Ausgangszustand versetzt wird, wobei das Inkontaktbringen in dem Gefäß (10) bei einem Sauerstoffpartialdruck unter 1 bar erfolgt, wobei zur Oxidation des Katalysators ein Teil der flüssigen Lösung (12) aus dem Gefäß (10) ausgeleitet, mit Sauerstoff oder einem Sauerstoff enthaltenden Gasgemisch bei einem Sauerstoffpartialdruck von 1 bis 500 bar beaufschlagt und anschließend wieder dem Rest der flüssigen Lösung (12) zugeführt wird, wobei der Sauerstoffpartialdruck in dem Gefäß stets unter 1 bar gehalten wird, wobei der den Teil der flüssigen Lösung (12) beaufschlagende Sauerstoffpartialdruck von 1 bis 500 bar auf unter 5 bar herabgesetzt wird, bevor der Teil der flüssigen Lösung (12) wieder dem Rest der flüssigen Lösung (12) zugeführt wird.

2. Verfahren nach Anspruch 1, wobei das alpha-Hydroxyaldehyd, die alpha-Hydroxycarbonsäure, das Kohlenhydrat, das Glycosid oder das Polymer in der flüssigen Lösung (12) in Form von darin verteilten Feststoffen vorliegt.

3. Verfahren nach einem der vorhergehenden Ansprüche, wobei das Polymer ein Polyester, ein Polyamin oder ein Polyamid, insbesondere Polyhexamethylenadipinsäureamid, ist.

4. Verfahren nach einem der vorhergehenden Ansprüche, wobei das Inkontaktbringen in dem Gefäß (10) bei Atmosphärendruck erfolgt, wobei der Gasdruck in dem Gefäß stets bei Atmosphärendruck gehalten wird.

5. Verfahren nach einem der vorhergehenden Ansprüche, wobei der Teil der flüssigen Lösung (12) kontinuierlich aus dem Gefäß (10) ausgeleitet, bei einem Sauerstoffpartialdruck von 1 bis 500 bar oxidiert und anschließend wieder dem Rest der flüssigen Lösung (12) kontinuierlich zugeführt wird.

6. Verfahren nach Anspruch 4, wobei das Oxidieren kontinuierlich durchgeführt wird.

7. Verfahren nach einem der vorhergehenden Ansprüche, wobei der Teil der flüssigen Lösung (12) vor dem Beaufschlagen mit dem Sauerstoff oder dem Gasgemisch abgekühlt wird und dem Sauerstoffpartialdruck von 1 bis 500 bar bei einer Temperatur von höchstens 95°C, höchstens 85°C, höchstens 75°C, höchstens 65°C, höchstens 55°C, höchstens 45°C, höchstens 35°C oder höchstens 25°C ausgesetzt wird.

8. Verfahren nach einem der vorhergehenden Ansprüche, wobei der Teil der flüssigen Lösung (12) mittels mindestens einer Pumpe oder Dickstoffpumpe gefördert wird.

9. Verfahren nach einem der vorhergehenden Ansprüche, wobei der Teil der flüssigen Lösung (12) dem Sauerstoffpartialdruck von 1 bis 500 bar in einem weiteren Gefäß ausgesetzt wird.

10. Verfahren nach Anspruch 9, wobei der Sauerstoff oder das Gasgemisch dem Teil der flüssigen Lösung (12) zugeführt wird, das weitere Gefäß ein Gehäuse (18) eines Schneckenverdichters ist und der Sauerstoffpartialdruck dadurch aufgebaut wird, dass der Teil der flüssigen Lösung (12) zusammen mit dem Sauerstoff oder dem Gasgemisch mittels des Schneckenverdichters verdichtet wird.

11. Verfahren nach einem der vorhergehenden Ansprüche, wobei der den Teil der flüssigen Lösung (12) beaufschlagende Sauerstoffpartialdruck von 1 bis 500 bar auf unter 1 bar oder auf den bei Atmosphärendruck vorliegenden Sauerstoffpartialdruck herabgesetzt wird, bevor der Teil der flüssigen Lösung (12) wieder dem Rest der flüssigen Lösung (12) zugeführt wird.

12. Verfahren nach einem der vorhergehenden Ansprüche, wobei bei oder nach der Herabsetzung des den Teil der flüssigen Lösung (12) beaufschlagenden Sauerstoffpartialdrucks oder nachdem der Teil der flüssigen Lösung (12) wieder dem Rest der flüssigen Lösung (12) zugeführt worden ist Gas (29), welches aus dem Teil der flüssigen Lösung (12) oder der flüssigen Lösung (12) ausgast, aus einer zur Durchführung des Verfahrens geeigneten Vorrichtung entweichen gelassen oder abgeführt wird.

13. Verfahren nach einem der vorhergehenden Ansprüche, wobei das Polymer ein Polymer ohne Weichmacher ist.

## Claims

1. A method for the catalytic generation of formic acid at an oxygen partial pressure below 1 bar and regeneration of the catalyst used therefor, wherein a polyoxometalate ion, serving as a catalyst, of general formula [PMoₓV_{y}O₄₀]ⁿ⁻ is brought into contact, at a temperature above 70°C and below 120°C, with an alpha-hydroxy aldehyde, an alpha-hydroxycarboxylic acid, a carbohydrate, a glycoside or a polymer containing a carbon chain and having at least one OH group bonded to the carbon chain repeatedly as a substituent and/or having an O, N or S atom contained repeatedly in said carbon chain, in a liquid solution (12) in a vessel (10), wherein 6 ≤ x ≤ 11 and 1 ≤ y ≤ 6 and 3 < n < 10 and x + y = 12, wherein each of n, x and y is an integer, wherein said catalyst reduced in this way is returned to its starting state by oxidation, wherein the contacting is carried out in said vessel (10) at an oxygen partial pressure below 1 bar, wherein, to oxidize said catalyst, a portion of said liquid solution (12) is diverted out of said vessel (10), has oxygen or a gas mixture containing oxygen applied thereto at an oxygen partial pressure of 1 to 500 bar, and is subsequently fed back to the rest of said liquid solution (12), wherein said oxygen partial pressure in said vessel is constantly maintained below 1 bar, wherein said oxygen partial pressure of 1 to 500 bar being applied to said portion of said liquid solution (12) is reduced to below 5 bar before said portion of said liquid solution (12) is fed back to said rest of said liquid solution (12).

2. The method according to claim 1, wherein said alpha-hydroxy aldehyde, said alpha-hydroxycarboxylic acid, said carbohydrate, said glycoside or said polymer is present in said liquid solution (12) in the form of solids distributed therein.

3. The method according to any one of the preceding claims, wherein said polymer is a polyester, a polyamine or a polyamide, in particular poly(hexamethylene adipamide).

4. The method according to any one of the preceding claims, wherein said contacting is carried out in the vessel (10) at atmospheric pressure, wherein the gas pressure in said vessel is constantly maintained at atmospheric pressure.

5. The method according to any one of the preceding claims, wherein said portion of said liquid solution (12) is continuously diverted out of said vessel (10), oxidized at an oxygen partial pressure of 1 to 500 bar and subsequently continuously fed back to the rest of said liquid solution (12).

6. The method according to claim 4, wherein said oxidation is carried out continuously.

7. The method according to any one of the preceding claims, wherein said portion of said liquid solution (12) is cooled before having said oxygen or said gas mixture applied thereto, and said portion is exposed to said oxygen partial pressure of 1 to 500 bar at a temperature of at most 95°C, at most 85°C, at most 75°C, at most 65°C, at most 55°C, at most 45°C, at most 35°C or at most 25°C.

8. The method according to any one of the preceding claims, wherein said portion of said liquid solution (12) is conveyed by means of at least one pump or slurry pump.

9. The method according to any one of the preceding claims, wherein said portion of said liquid solution (12) is exposed to said oxygen partial pressure of 1 to 500 bar in a further vessel.

10. The method according to claim 9, wherein said oxygen or said gas mixture is fed to said portion of said liquid solution (12), said further vessel is a housing (18) of a screw compressor and said oxygen partial pressure is built up in that said portion of said liquid solution (12) is compressed, together with said oxygen or said gas mixture, by means of said screw compressor.

11. The method according to any one of the preceding claims, wherein said oxygen partial pressure of 1 to 500 bar being applied to said portion of said liquid solution (12) is reduced to below 1 bar or to the oxygen partial pressure present at atmospheric pressure, before said portion of said liquid solution (12) is fed back to said rest of said liquid solution (12).

12. The method according to any one of the preceding claims, wherein, during or after the reduction of said oxygen partial pressure being applied to said portion of said liquid solution (12), or after said portion of said liquid solution (12) has been fed back to said rest of said liquid solution (12), gas (29), which gases out from said portion of said liquid solution (12) or said liquid solution (12), is allowed to escape or is discharged from a device suitable for carrying out said method.

13. The method according to any one of the preceding claims, wherein said polymer is a polymer without plasticizer.

## Revendications

1. Procédé de génération catalytique d'acide formique à une pression partielle d'oxygène en dessous de 1 bar et de régénération du catalyseur utilisé ce faisant, dans lequel un ion polyoxométallate servant de catalyseur de la formule générale [PMoₓV_{y}O₄₀]ⁿ⁻ est amené en contact à une température au-dessus de 70°C et en dessous de 120°C avec un alpha-hydroxyaldéhyde, un acide alpha-hydroxycarbonique, un hydrate de carbone, un glycoside ou un polymère contenant une chaîne carbonée avec au moins un groupe OH lié de manière répétée en tant que substituant à la chaîne carbonée et/ou avec un atome de O, N ou S contenu de manière répétée dans la chaîne carbonée dans une solution liquide (12) dans un récipient (10), dans laquelle 6 ≤ x ≤ 11 et 1 ≤ y ≤ 6 et 3 < n < 10 et x + y = 12, dans lequel n, x et y sont chacun un nombre entier, dans lequel le catalyseur réduit ce faisant est remis dans son état de départ par oxydation, dans lequel la mise en contact dans le récipient (10) s'effectue à une pression partielle d'oxygène en dessous de 1 bar, dans lequel une partie de la solution liquide (12) est déviée du récipient (10) pour l'oxydation du catalyseur, pressurisée avec de l'oxygène ou un mélange gazeux contenant de l'oxygène à une pression partielle d'oxygène de 1 à 500 bars, puis de nouveau amenée au reste de la solution liquide (12), dans lequel la pression partielle d'oxygène dans le récipient est toujours maintenue en dessous de 1 bar, dans lequel la pression partielle d'oxygène pressurisant la partie de la solution liquide (12) est abaissée de 1 à 500 bars à moins de 5 bars avant que la partie de la solution liquide (12) ne soit de nouveau amenée au reste de la solution liquide (12).

2. Procédé selon la revendication 1, dans lequel l'alpha-hydroxyaldéhyde, l'acide alpha-hydroxycarbonique, l'hydrate de carbone, le glycoside ou le polymère dans la solution liquide (12) est présent sous forme de solides répartis en son sein.

3. Procédé selon une des revendications précédentes, dans lequel le polymère est un polyester, une polyamine ou un polyamide, notamment le polyhexaméthylène adipamide.

4. Procédé selon une des revendications précédentes, dans lequel la mise en contact dans le récipient (10) s'effectue à la pression atmosphérique, dans lequel la pression gazeuse dans le récipient est toujours maintenue à la pression atmosphérique.

5. Procédé selon une des revendications précédentes, dans lequel la partie de la solution liquide (12) est déviée en continu du récipient (10), oxydée à une pression partielle d'oxygène de 1 à 500 bars, puis de nouveau amenée en continu au reste de la solution liquide (12).

6. Procédé selon la revendication 4, dans lequel l'oxydation est réalisée en continu.

7. Procédé selon une des revendications précédentes, dans lequel la partie de la solution liquide (12) est refroidie avant la pressurisation avec l'oxygène ou le mélange gazeux et est exposée à la pression partielle d'oxygène de 1 à 500 bars à une température de 95°C maximum, 85°C maximum, 75°C maximum, 65°C maximum, 55°C maximum, 45°C maximum, 35°C maximum ou 25°C maximum.

8. Procédé selon une des revendications précédentes, dans lequel la partie de la solution liquide (12) est transportée au moyen d'au moins une pompe ou pompe à liquides épais.

9. Procédé selon une des revendications précédentes, dans lequel la partie de la solution liquide (12) est exposée à la pression partielle d'oxygène de 1 à 500 bars dans un autre récipient.

10. Procédé selon la revendication 9, dans lequel l'oxygène ou le mélange gazeux est amené à la partie de la solution liquide (12), l'autre récipient est un logement (18) d'un compresseur à vis sans fin et la pression partielle d'oxygène est constituée en ce que la partie de la solution liquide (12) est comprimée avec l'oxygène ou le mélange gazeux au moyen du compresseur à vis sans fin.

11. Procédé selon une des revendications précédentes, dans lequel la pression partielle d'oxygène pressurisant la partie de la solution liquide (12) est abaissée de 1 à 500 bars à moins de 1 bar ou à la pression partielle d'oxygène présente à la pression atmosphérique avant que la partie de la solution liquide (12) ne soit de nouveau amenée au reste de la solution liquide (12).

12. Procédé selon une des revendications précédentes, dans lequel lors de ou après l'abaissement de la pression partielle d'oxygène pressurisant la partie de la solution liquide (12) ou après que la partie de la solution liquide (12) a de nouveau été amenée au reste de la solution liquide (12), du gaz (29), qui est dégazé de la partie de la solution liquide (12) ou de la solution liquide (12), est laissé s'échapper ou évacué d'un dispositif convenant à la réalisation du procédé.

13. Procédé selon une des revendications précédentes, dans lequel le polymère est un polymère sans plastifiant.
